# EUROPEAN PATENT SPECIFICATION

(11) **EP 0 519 983 B1**
(45) Date of publication and mention of the grant of the patent: **15.05.1996**
(21) Application number: 91906055.8
(22) Date of filing: 01.03.1991
(51) Int. Cl.: A61K 31/40

(54) **USE OF KETOROLAC FOR TREATMENT OF PERIODONTAL DISEASE**
VERWENDUNG VON KETOROLAC ZUR BEHANDLUNG VON PERIODONTITIS
UTILISATION DE CETOROLAC POUR LE TRAITEMENT DE LA PARADONTOLYSE

(30) Priority: 16.03.1990 US 494697; 08.02.1991 US 651061
(43) Date of publication of application: 30.12.1992
(73) Proprietor: THE PROCTER & GAMBLE COMPANY, Cincinnati, Ohio 45202 (US)
(72) Inventor: KELM, Gary, Robert, Cincinnati, OH 45247 (US); PICKRUM, Harvey, Marvin, Cincinnati, OH 45231 (US); DOYLE, Matthew, Joseph, Cincinnati, OH 45224 (US)
(74) Representative: Brooks, Maxim Courtney
(86) International application number: US9101441
(87) International publication number: WO9113609

(56) References cited:
- EP-A- 0 306 984
- EP-A- 0 380 367
- GB-A- 1 550 139
- US-A- 4 919 939
- JOURNAL OF PHARMACEUTICAL SCIENCES, vol. 76, no. 11, November 1987, page S280; W.C. LIAW et al.: "Percutaneous absorption of ketorolac topical gel formulations: vehicle effects and in vitro-in vivo correlations"
- JOURNAL OF MEDICINAL CHEMISTRY, vol. 29, no. 4, April 1986, pages 589-591, American Chemical Society; A. GUZMAN et al.: "Absolute configuration of (-)-5- benzoyl-1,2-dihydro-3H-pyrrolo(1,2-alpa)pyrrole-1-carboxylic acid, the active enantiomer of ketorolac"

## Description

The present invention relates to the use of ketorolac in the manufacture of compositions for the treatment of periodontal disease.

"Periodontal disease", as used herein, is a broad term used to describe those diseases which attack the gingiva and the underlying alveolar bone supporting the teeth. Periodontal disease includes a series of diseases exhibiting various syndromes which vary from each other according to the stage or situation of the disease or the age of the patient, and have not been definitely subclassified. The term is used for any inflammatory disease which initially occurs at a marginal gingiva area and may affect the alveolar bone. Two common periodontal diseases are gingivitis (inflammation of the gingiva) and periodontitis (manifested by progressive resorption of alveolar bone, increasing mobility of the teeth, and loss of the teeth at advanced stage). Other terms used for various aspects of periodontal disease include "juvenile periodontitis", "acute necrotizing ulcertive gingivitis", and "alveolar pyorrhea". Periodontal disease is characterized by one or more of the following: inflammation of the gingiva, formation of periodontal pockets, bleeding and/or pus discharge from the periodontal pockets, resorption of alveolar bone, loose teeth and loss of teeth.

Periodontal disease is generally considered to be caused by/associated with bacteria which are generally present in dental plaque which forms on the surface of the teeth and in the periodontal pocket. Known methods for treating periodontal disease often include the use of antimicrobials.

Certain nonsteroidal anti-inflammatory drugs (NSAIDs) have been shown to be useful in the treatment of periodontal disease, as disclosed in the following references: EP-A-0,137,668; Waite, I.M., C.A. Saxton, A. Young, B.J. Wagg & M. Corbett, "The Periodontal Status of Subjects Receiving Non-Steroidal Anti-Inflammatory Drugs", Journal of Periodontal Research, Vol. 16 (1981), pp. 100-108; Feldman, R.S., B. Szeto, H.J. Chauncey & P. Goldhaber, "Non-Steroidal Anti-Inflammatory Drugs in the Reduction of Human Alveolar Bone Loss", Journal of Clinical Periodontology, Vol. 10, No. 2 (1983), pp. 131-136; Vogel, R.I., S.A. Copper, L.G. Schneider & D. Goteiner, "The Effects of Topical Steroidal and Systemic Nonsteroidal Anti-Inflammatory Drugs on Experimental Gingivitis in Man", Journal of Periodontology, Vol. 55, No. 4 (1984), pp. 247-251; Vogel, R.I., "The Experimental Use of Anti-Inflammatory Drugs in the Treatment of Periodontal Disease:, Journal of Periodontology, Vol 56 (suppl) (1985), pp. 88-92; Vogel, R.I., L. Schneider & D. Goteiner, "The Effects of a Topically-Active Non-Steroidal Anti-Inflammatory Drug on Ligature-Induced Periodontal Disease in the Squirrel Monkey", Journal of Clinical Periodontology, Vol. 13 (1986), pp. 139-144; Williams, R.C., "Non-Steroidal Anti-Inflammatory Drugs in Periodontal Disease", Non-Steroidal Anti-Inflammatory Drugs, A.J. Lewis, D.E. Furst, eds., Marcel Dekker, pub., (1987), pp. 143-155; Williams, R.C., M.K. Jeffcoat, T.H. Howell, C.M. Hall, H.G. Johnson, W.J. Wechter & P. Goldhaber, "Indomethacin or Flurbiprofen Treatment of Periodontitis in Beagles: Comparison of Effect on Bone Loss", Journal of Periodontal Research, Vol. 22 (1987), pp. 403-407; Rieger, M.M., "Topical Antiinflammatory Therapy Against Periodontal Disease: A Historical Survey", Clinical Preventive Dentistry, Vol. 9, No. 4 (1987), pp. 18-22; Williams, R.C., M.K. Jeffcoat, T.H. Howell, M.S. Reddy, H.G. Johnson, C.M. Hall & P. Goldhaber, "Topical Flurbiprofen Treatment of Periodontitis in Beagles", Journal of Periodontal Research, Vol. 23 (1988), pp. 166-169; Jeffcoat, M.K., R.C. Williams, M.S. Reddy, R. English & P. Goldhaber, "Flurbiprofen Treatment of Human Periodontitis: Effect on Alveolar Bone Height and Metabolism", Journal of Periodontal Research, Vol. 23 (1988), pp. 381-385; Williams, R.C., M.K. Jeffcoat, T.H. Howell, M.S. Reddy, H.G. Johnson, C.M. Hall & P. Goldhaber, "Ibuprofen: An Inhibitor of Alveolar Bone Loss in Beagles", Journal of Periodontal Research, Vol. 23 (1988), pp. 225-229; Heasman, P.A., R.A. Seymour & P.F. Boston, "The Effect of a Topical Non-Steroidal Anti-Inflammatory Drug on the Development of Experimental Gingivitis in Man", Journal of Clinical Periodontology, Vol. 16 (1989), pp. 353-358; Williams, R., M. Jeffcoat, H. Howell, M. Reddy, C. Hall, H. Johnson & P. Goldhaber, "Naproxen Treatment of Periodontitis in Beagles", Journal of Dental Research, (1989), p. 243 (Abstract No. 491); Gaffar, A., J. Afflitto, E.J. Coleman, L. Steinberg & D. Fine, "Efficacy of Ibuprofen Rinse in a Sub-gingival Irrigator on Periodontitis in Primates", Journal of Dental Research, Vol. 68 (Special Issue) (1989), Abstract No. 830; and Williams, R.C., M.K. Jeffcoat, T.H. Howell, A. Rolla, D. Stubbs, K.W. Teoh, M.S. Reddy & P. Goldhaber, "Altering the Progression of Human Alveolar Bone Loss with the Non-Steroidal Anti-Inflammatory Drug Flurbiprofen, Journal of Periodontology, Vol. 60 (1989), pp. 485-490.

Ketorolac and its pharmaceutically-acceptable, non-toxic esters and salts are known NSAIDs. Ketorolac and its esters and salts, particularly its tromethamine salt, are disclosed in the following references: U.S. Patent No. 4,089,969 issued to Muchowski & Kluge on May 16, 1978; Rooks, W.H., P.J. Maloney, L.D. Shott, M.E. Schuler, H. Sevelius, A.N. Strosberg, L. Tanenbaum, A.J. Tomolonis, M.B. Wallich, D. Waterbury & J.P. Yee, "The Analgesic and Anti-Inflammatory Profile of Ketorolac and its Tromethamine Salt", Drugs Experimental Clinical Research, Vol. XI, No. 8 (1985), pp. 479-492; and Mroszcsak, E.J., F.W. Lee, D. Combs, F.H. Sarnquist, B-L Huang, A.T. Wu, L.G. Tokes, M.L. Maddox & D.K. Cho, "Ketorolac Tromethamine Absorption, Distribution, Metabolism, Excretion, and Pharmacokinetics in Animals and Humans", Drug Metabolism and Disposition, Vol. 15, No. 5 (1987), pp. 618-626.

Several NSAIDs (e.g., ibuprofen and naproxen) are known to have more than one enantiomeric form which differ in properties from one another. Advantages of certain enantiomeric forms of NSAIDs are disclosed in the following references: PCT Patent Application No. WO/00421 of Sunshine & Laska, published January 26, 1989; Caldwell, J., A.J. Hutt & S. Fournel-Gigleux, "The Metabolic Chiral Inversion and Disposition Enantioselectivity of the 2-Arylpropionic Acids and Their Biological Consequences", Biochemical Pharmacology, Vol. 37 (1988), pp. 105-114; and Hutt, A.J. & J. Caldwell, "The Metabolic Chiral Inversion of 2-Aryl-propionic Acids - A Novel Route with Pharmacologic Consequences", Journal of Pharmacy and Pharmacology, Vol. 35 (1983), pp. 693-704.

It is an object of the present invention to provide a medicament for topical, oral treatment of periodontal disease using ketorolac.

The present invention relates to the use of ketorolac for the manufacture of a topical oral medicament for prevention or treatment of periodontal disease.

"Ketorolac", as used herein, is (±)-5-benzoyl-2,3-dihydro-1H-pyrrolizine-1-carboxylic acid, and the pharmaceutically-acceptable non-toxic esters and salts thereof, as disclosed in U.S.-A-4,089,969. The (-) or S enantiomer of ketorolac is preferred.

Pharmaceutically-acceptable esters of ketorolac include, but are not limited to, alkyl esters derived from hydrocarbons of branched or straight chain having one to about 12 carbon atoms. Examples of such esters are methyl, ethyl, propyl, isopropyl, butyl, t-butyl, isoamyl, pentyl, isopentyl, hexyl, octyl, nonyl, isodecyl, 6-methyldecyl and dodecyl esters.

Pharmaceutically-acceptable salts of ketorolac include salts derived from either inorganic or organic bases. Salts derived from inorganic bases include sodium, potassium, lithium, ammonium, calcium, magnesium, ferrous, zinc, copper, manganous, aluminum, ferric, manganic salts and the like. Particularly preferred are the ammonium, potassium, sodium, and lithium salts. Salts derived from pharmaceutically-acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, piperidine, tromethamine, dicyclohexylamine, choline and caffeine.

A suitable salt of ketorolac is soluble in the composition of the subject invention in which it is incorporated. The preferred ketorolac salt for use in the compositions and methods of the present invention is ketorolac tromethamine, especially its (-) or S enantiomer, (-)5-benzoyl-2,3-dihydro-1H-pyrrolizine-1-carboxylic acid, 2-amino-2-(hydroxymethyl)-1,3-propanediol:

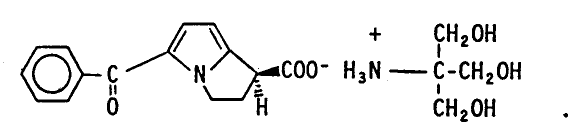

The compositions herein comprise a safe and effective amount, preferably from 0.001% to 5%, more preferably from 0.005% to 1%, more preferably still from 0.01% to 0.5%, still more preferably from 0.05% to 0.2% ketorolac, and a pharmaceutically-acceptable topical, oral carrier. Also preferred are compositions comprising less than 0.15% ketorolac, and also those comprising less than 0.025% ketorolac.

The pH of the compositions of the present invention for which pH can be measured is preferably from 2 to 9, more preferably from 4 to 7, more preferably still from 5 to 6.

"Safe and effective amount", as used herein, means an amount of a substance high enough to provide a significant positive modification of the condition to be treated, but low enough to avoid serious side effects (at a reasonable benefit/risk ratio), within the scope of sound medical judgement. A safe and effective amount of the substance will vary with the particular condition being treated, the severity of the condition, the duration of the treatment, the nature of concurrent therapy, and like factors.

"Topical, oral carrier", as used herein, denotes a carrier for the ketorolac which results in a composition which is administered topically to the oral cavity, held therein for a period of time, and then is largely expectorated rather than being swallowed. Such compositions include toothpastes, tooth gels, tooth powders, mouthwashes, mouthsprays, prophylaxis pastes, dental treatment solutions, biogels or other sustained release products, and the like.

Components of the topical, oral carrier are suitable for administration to the oral cavity of a human or lower animal and are compatible with one another and the other components, especially the ketorolac, used in an oral composition of the present invention. The term "compatible" as used herein, means that the components of the compositions are capable of being commingled with one another, in a manner such that there is no interaction which would substantially reduce the efficacy of the oral composition under ordinary use conditions. Preferred topical, oral carriers thus provide the desired characteristics for toothpastes, tooth gels, tooth powders, mouthwashes, mouthsprays, prophylaxis pastes, dental treatment solutions, and the like. The topical, oral carriers comprise components typically used in such compositions which are well known to a skilled practitioner. Such components include, but are not limited to anticaries agents, antiplaque agents, anticalculus agents, dental abrasives, surfactants, flavoring agents, sweetening agents, binders, humectants, thickening agents, buffering agents, preservatives, coloring agents and pigments, ethanol and water.

Water is an optional component of the topical, oral carriers of the compositions of the present invention. Water employed in the preparation of the commercially suitable compositions should preferably be of low ion content and free of organic impurities. Water preferably comprises from 2% to 99%, more preferably from 20% to 95% of the compositions of the present invention. When in the form of toothpastes, the compositions preferably are from 2% to 45%, more preferably from 30% to 40%, water, while mouthwashes are preferably from 45% to 95%, more preferably from 75% to 90%, water.

Dental abrasives useful in the topical, oral carriers of the compositions of the present invention include many different materials. The material selected must be one which is compatible within the composition of interest and does not excessively abrade dentin. Suitable abrasives include, for example, silicas including gels and precipitates, insoluble sodium polymetaphosphate, hydrated alumina, and resinous abrasive materials.

A class of preferred abrasives for use in the subject compositions is the particulate thermo-setting polymerized resins as described in U.S.-A-3,070,510. Suitable resins include, for example, melamines, phenolics, ureas, melamine-ureas, melamine-formaldehydes, urea-formaldehydes, melamine-urea-formaldehydes, cross-linked epoxides, and cross-linked polyesters.

Silica dental abrasives are also preferred in the compositions of the present invention. The silica abrasive polishing material generally has an average particle size ranging between 0.1 and 30 microns, preferably between 5 and 15 microns. The abrasive can be precipitated silica or silica gels such as the silica xerogels described in U.S.-A-3,538,230 and in U.S.-A-3,862,307. Preferred are the silica xerogels marketed under the tradename Syloid® by the W. R. Grace & Co., Davison Chemical Division. Preferred precipitated silica materials are those marketed by the J.M. Huber Corporation under the tradename Zeodent®, particularly the silica carrying the designation Zeodent 119®. These silica abrasives are described in U.S.-A-4,340,583.

Mixtures of abrasives can be used.

The total amount of abrasive in dentifrice compositions of the present invention preferably range from 10% to 70% by weight; toothpastes preferably contain from 10% to 50% by weight of abrasives. Solution, mouthspray and mouthwash compositions of the present invention may contain quantities of abrasive as low as 0%.

Flavoring agents are preferred in the topical, oral carriers of the compositions of the present invention in order to make them more palatable. Typical flavoring agents include methanol, oil of wintergreen, oil of peppermint, oil of spearmint, oil of sassafras, and oil of clove. If present, flavoring agents are generally included in the subject compositions in amounts of from 0.04% to 2% by weight.

Sweetening agents are also preferred in the topical, oral carriers of the compositions of the present invention in order to make them more palatable. Typical sweetening agents include saccharin salts, dextrose, levulose, thaumatin, aspartame, D-tryptophan, dihydrochalcones, acesulfame and cyclamate salts, especially sodium cyclamate and sodium saccharin. If present, sweetening agents are generally included in the subject compositions in amounts of from 0.01% to 5% by weight.

Another optional component of the topical, oral carriers of the compositions of the present invention is a humectant. The humectant serves to keep toothpaste compositions from hardening upon exposure to air, and to give mouthwash and toothpaste compositions a moist feel to the mouth. Certain humectants can also impart desirable sweetness of flavor to mouthwash and toothpaste compositions. The humectant, on a pure humectant basis, generally comprises from 0% to 70%, preferably from 2% to 55%, by weight of the compositions herein. Suitable humectants for use in compositions of the present invention include edible polyhydric alcohols such as glycerin, sorbitol, xylitol, polyethylene glycol, and propylene glycol, especially sorbitoland glycerin.

Buffering agents are another optional component of the topical, oral carrier of the compositions of the present invention. The buffering agents serve to retain the pH of the compositions within the preferred range. The buffering agent generally comprises from 0% to 10%, preferably from 0.2% to 5%, by weight of the compositions herein. Suitable buffering agents for use in compositions of the present invention include soluble phosphate salts.

Other optional components of the topical, oral carriers of the compositions of the present invention are preservatives. The preservatives prevent microbial growth in the compositions. Suitable preservatives include methylparaben, propylparaben, benzoates and ethanol. If the preservative is ethanol, it generally comprises from 0% to 35%, preferably from 5% to 15%, of the compositions herein. Other preservatives generally comprise from 0% to 5%, preferably from 0.1% to 2%, by weight of the compositions herein.

Binders and thickening agents may be used in the topical, oral carriers of the compositions of the present invention, particularly in toothpaste compositions. Preferred binders and thickening agents include, for example, carrageenan (e.g., Irish moss, Viscarin TP-5 which is an iota carrageenan), cellulose derivatives (e.g., hydroxyethyl cellulose, sodium carboxymethyl cellulose, sodium carboxymethyl hydroxypropyl cellulose), carboxyvinyl polymers (carbomers), natural gums (e.g., gum karaya, gum arabic, gum tragacanth), polysaccharide gums (e.g., xanthan gum), fumed silica, and colloidal magnesium aluminum silicate. If present, these binders and thickening agents are generally present in the compositions of the present invention in amounts of from 0.1% to 5%.

Compositions of the present invention may also contain a surfactant. Suitable surfactants are those which are reasonably stable and preferably form suds through the pH range of the compositions. Surfactants useful as sudsing agents may be soaps, and anionic, nonionic, cationic, zwitterionic and amphoteric organic synthetic detergents, and compatible mixtures thereof. Surfactants of these types are described more fully in U.S.-A-3,959,458. Such surfactants are generally present in the compositions of the present invention at a level of from 0% to 10%, preferably from 0.2% to 5%. Surfactants may also be used as solubilizing agents to help retain sparingly soluble components, e.g., some flavoring agents, in solutions. Surfactants suitable for this purpose include polysorbates and poloxamers.

The compositions of the present invention may also comprise an anticaries agent. Preferred anticaries agents are water-soluble fluoride ion sources. Fluoride ions also generally help stabilize pyrophosphate (generally an anticalculus agent) in the oral cavity, thus enhancing the benefits provided by any soluble pyrophosphate included in the compositions. The number of such fluoride ion sources is great and includes those disclosed in U.S.-A-3,535,421. Preferred fluoride ion source materials include: sodium fluoride, potassium fluoride, and sodium monofluorophosphate and mixtures thereof. Sodium fluoride is the preferred fluoride source. The amount of the fluoride ion source in the oral compositions of the present invention, if present, is preferably sufficient to provide from 0.005% to 0.35%, more preferably from 0.05% to 0.3% of fluoride ions in the compositions.

Antimicrobial antiplaque agents can also optionally be present in the oral compositions of the present invention, on the condition that they are compatible with the ketorolac. Such agents may include Triclosan, 5-chloro-2-(2,4-dichlorophenoxy)-phenol, as described in The Merck Index, 10th ed. (1976), p. 1381; U.S.-A-3,506,720; and EP-A-0,251,591. If present, the antimicrobial antiplaque agents generally comprise from 0.1% to 5% by weight of the compositions of the present invention.

Compositions of the present invention may also include one or more anticalculus agents, on the condition that they are compatible with the ketorolac. Anticalculus agents which may be useful in the compositions of the present invention include pyrophosphates or polyphosphates such as those disclosed in U.S.-A-4,590,066; polyacrylates and other polycarboxylates such as those disclosed in U.S.-A-3,429,963 and U.S.-A-4,304,766; and U.S.-A-4,661,341; polyepoxysuccinates such as those disclosed in U.S.-A-4,846,650; ethylenediaminetetraacetic acid as disclosed in GB-A-490,384; nitrilotriacetic acid and related compounds as disclosed in U.S.-A-3,678,154; polyphosphonates as disclosed in U.S.-A-3,737,533, U.S.-A-3,988,443 and U.S.-A-4,877,603. If present, the anticalculus agents generally comprise from 0.2% to 13%, preferably from 0.4% to 6% of the compositions of the present invention.

Preferred compositions of the present invention are in the form of toothpastes. Components of such toothpastes generally include a dental abrasive (from 10% to 50%), a surfactant (from 0.5% to 10%), a thickening agent (from 0.1% to 5%), a humectant (from 10% to 55%), a flavoring agent (from 0.04% to 2%), a sweetening agent (from 0.1% to 3%), a coloring agent (from 0.01% to 0.5%) and water (from 2% to 45%). Such toothpastes may also include one or more of an anticaries agent (from 0.05% to 0.3% as fluoride ion), an anticalculus agent (from 0.1% to 13%), and an antiplaque agent (from 0.1% to 5%).

Other preferred compositions of the present invention are mouthwashes and mouthsprays. Components of such mouthwashes and mouthsprays include water (from 45% to 95%), ethanol (from 0% to 25%), a humectant (from 0% to 50%), a surfactant agent (from 0.01% to 7%), an flavoring agent (from 0.04% to 2%), a sweetening agent (from 0.1% to 3%), and a coloring agent (from 0.001% to 0.5%). Such mouthwashes and mouthsprays may also include one or more of an anticaries agent (from 0.05% to 0.3% as fluoride ion), an anticalculus agent (from 0.1% to 3%), and an antiplaque agent (from 0.1% to 5%).

Other preferred compositions of the present invention are dental solutions. Components of such dental solutions generally include water (from 90% to 99%), preservative (from 0.01% to 0.5%, thickening agent (from 0% to about 5%), flavoring agent (from 0.04% to 2%), sweetening agent (from 0.1% to 3%), and surfactant (from 0% to 5%).

The compositions of the invention are used for treating periodontal disease by topical application, to gingival mucosa afflicted with the disease.

It has unexpectedly been found that, whereas the use of other nonsteroidal anti-inflammatory drugs for the topical treatment of periodontal disease in the oral cavity has resulted in substantial systemic concentrations of the drug, the systemic concentration of ketorolac resulting from the methods of the present invention is very low, as long as swallowing of the composition is substantially avoided. While the systemic concentration of ketorolac resulting from its topical application is very low, a substantial amount of the ketorolac is found in the crevicular fluid, which flows from the gingival tissues.

The treatment of periodontal disease by the topical application of ketorolac not only reduces inflammation caused by the disease, but also unexpectedly reduces the rate of alveolar bone resorption (bone loss) characteristic of periodontitis.

In use, the compositions of the present invention are contacted with oral cavity tissue afflicted with periodontal disease for at least about 15 seconds, preferably from about 20 seconds to about 10 minutes, more preferably from about 30 seconds to about 60 seconds.

The following examples are provided as illustrations of the composition of the present invention.

### Examples 1 and 2

Examples of toothpaste compositions of the present invention are as follows:

| Ingredients | Example 1 (Wt. %) | Example 2 (Wt. %) |
|---|---|---|
| Sorbitol | 42.00 | 35.00 |
| Saccharin Sodium | 0.13 | 0.20 |
| FD&C Blue (1% soln) | 0.05 | 0.05 |
| Precipitated Silica | 20.00 | 25.00 |
| Sodium Fluoride | ---- | 0.24 |
| Flavor | 0.90 | 1.50 |
| Purified Water | qs | qs |
| Sodium Alkyl Sulfate | 1.00 | 1.20 |
| Phosphoric Acid | 0.40 | ---- |
| Carbomer 940 | 0.25 | 0.25 |
| Xanthan Gum | 0.50 | 0.65 |
| Titanium Dioxide | 0.50 | 0.50 |
| Ketorolac Tromethamine | 0.05 | 0.10 |

### Examples 3 and 4

Examples of mouthwash compositions of the present invention are as follows:

| Ingredients | Example 3 (Wt. %) | Example 4 (Wt. %) |
|---|---|---|
| Ketorolac Tromethamine | 0.10 | 0.01 |
| Ethanol | 12.00 | 15.00 |
| Glycerin | 10.00 | 12.00 |
| Dibasic Sodium Phosphate Heptahydrate | 0.07 | 0.48 |
| Saccharin Sodium | 0.08 | 0.08 |
| Monobasic Sodium Phosphate Monohydrate | 2.03 | 1.82 |
| Polysorbate 80 | 0.33 | 0.33 |
| FD&C Blue (1% Soln) | 0.02 | 0.02 |
| Flavor | 0.15 | 0.15 |
| Purified Water | qs | qs |

### Example 5

An example of a dental solution of the present invention is as follows:

| Ingredients | Example 5 (Wt. %) |
|---|---|
| Water | qs |
| Ketorolac Tromethamine | 0.15 |
| Flavor | 0.10 |
| Polysorbate 80 | 0.25 |
| Saccharin Sodium | 0.05 |
| Methylparaben | 0.20 |
| Propylparaben | 0.10 |

### Examples 6 and 7

Examples of toothpaste compositions of the present invention are as follows:

| Ingredients | Example 6 (Wt. %) | Example 7 (Wt. %) |
|---|---|---|
| Sorbitol | 37.20 | 37.20 |
| Glycerine | 19.00 | 19.00 |
| Polyethylene Glycol 600 | 3.00 | 3.00 |
| Sodium Saccharin | 0.17 | 0.17 |
| Precipitated Silica | 20.00 | 20.00 |
| Sodium Fluoride | 0.24 | 0.24 |
| Flavor | 0.90 | 0.90 |
| Purified Water | qs | qs |
| Sodium Alkyl Sulfate | 1.00 | 1.00 |
| Monobasic Sodium Phosphate, Monohydrate | 5.00 | 5.00 |
| Fumed Silica | 2.00 | 2.00 |
| Carboxymethylcellulose | 0.30 | 0.30 |
| Titanium Dioxide | 0.50 | 0.50 |
| Ketorolac Tromethamine | 0.15 | 1.00 |

## Claims

1. The use of ketorolac, preferably ketorolac tromethamine, for the manufacture of a topical, oral medicament to treat or prevent periodontal disease.

2. The use of Claim 1 wherein the medicament comprises from 0.001% to 1%, preferably from 0.01% to 0.5%, ketorolac.

3. A toothpaste composition comprising:
(a) from 0.001% to 1%, preferably from 0.01% to 0.5%, ketorolac; and
(b) a toothpaste carrier comprising a dental abrasive, a surfactant, a thickening agent, a humectant, a flavoring or sweetening agent, and water.

4. A mouthwash composition comprising:
(a) from 0.001% to 0.5%, preferably from 0.01% to 0.2%, ketorolac; and
(b) a mouthwash carrier comprising ethanol, a humectant, a surfactant, a flavoring or sweetening agent, and water.

5. A dental solution composition comprising:
(a) from 0.001% to 0.5%, preferably from 0.01% to 0.2%, ketorolac; and
(b) a dental solution carrier comprising a preservative, preferably a paraban preservative, and water.

6. The composition of any of Claims 2-5 wherein the composition has a pH of from 4 to 7.

7. The composition of any of Claims 2-6 wherein the ketorolac is ketorolac tromethamine.

8. The composition of any of Claims 2-7 wherein the ketorolac substantially consists of the (-) or S enantiomer.

9. The use of ketorolac according to Claim 1 or 2 wherein the medicament is in a form selected from toothpastes, tooth gels, tooth powders, mouthwasher, mouth sprays, prophalaxis pastes and dental treatment solutions.

10. The use of ketorolac according to Claim 1, 2 or 9 wherein the medicament is held in the oral cavity for a period of time end is then largely expectorated rather than being swallowed.

## Patentansprüche

1. Verwendung von Ketorolac, vorzugsweise Ketorolactromethamin, zur Herstellung eines topischen, oralen Medikaments zur Behandlung oder Verhütung von Periodontal-Erkrankungen.

2. Verwendung nach Anspruch 1, worin das Medikament von 0,001% bis 1%, vorzugsweise von 0.01% bis 0,5% Ketorolac umfaßt.

3. Zahnpastenzusammensetzung, umfassend:
(a) von 0,001% bis 1%, vorzugsweise von 0,1% bis 0,5% Ketorolac; und
(b) einen Zahnpastenträger, umfassend ein Dentalschleifmittel, ein grenzflächenaktives Mittel, ein Verdickungsmittel, ein Feuchthaltemittel, ein Aroma- oder Süßungsmittel und Wasser.

4. Mundwasserzusammensetzung, umfassend:
(a) von 0,001% bis 0,5%, vorzugsweise von 0,01% bis 0,2% Ketorolac; und
(b) einen Mundwasserträger, umfassend Ethanol, ein Feuchthaltemittel, ein grenzflächenaktives Mittel, ein Aroma- oder Süßungsmittel und Wasser.

5. Dentallösungszusammensetzung, umfassend:
(a) von 0,001% bis 0,5%, vorzugsweise von 0,01% bis 0,2% Ketorolac; und
(b) einen Dentallösungsträger, umfassend ein Konservierungsmittel, vorzugsweise ein Paraban-Konservierungsmittel, und Wasser.

6. Zusammensetzung nach einem der Ansprüche 2 bis 5, worin die Zusammensetzung einen pH-Wert von 4 bis 7 aufweist.

7. Zusammensetzung nach einem der Ansprüche 2 bis 6, worin das Ketorolac Ketorolac-tromethamin ist.

8. Zusammensetzung nach einem der Ansprüche 2 bis 7, worin das Ketorolac im wesentlichen aus dem (-)- oder S-Enantiomer besteht.

9. Verwendung von Ketorolac nach Anspruch 1 oder 2, worin das Medikament in einer Form vorliegt, ausgewählt unter Zahnpasten, Zahngelen, Zahnpulvern, Mundwässern, Mundsprühmitteln, Prophylaxe-Pasten und Dentalbehandlungslösungen.

10. Verwendung von Ketorolac nach Anspruch 1, 2 oder 9, worin das Medikament eine Zeit lang in der Mundhöhle gehalten und dann weitgehend ausgespuckt statt verschluckt wird.

## Revendications

1. Utilisation de kétorolac, de préférence de kétorolac-trométhamine, pour préparer un médicament topique par voie orale destiné au traitement ou à la prévention de la parodontolyse.

2. Utilisation selon la revendication 1, dans laquelle le médicament comprend de 0,001 % à 1 %, de préférence de 0,01 % à 0,5 % de kétorolac.

3. Composition de pâte dentifrice comprenant :
(a) de 0,001 % à 1 % et de préférence de 0,01 % à 0,5 % de kétorolac, et
(b) un excipient pour pâte dentifrice comprenant un abrasif dentaire, un tensioactif, un agent épaississant, un humectant, un aromatisant ou un édulcorant, et de l'eau.

4. Composition de bain de bouche comprenant :
(a) de 0,001 % à 0,5 % et de préférence de 0,01 % à 0,2 % de kétorolac, et
(b) un excipient pour bain de bouche comprenant de l'éthanol, un humectant, un tensioactif, un aromatisant ou un édulcorant, et de l'eau.

5. Composition de solution dentaire comprenant :
(a) de 0,001 % à 0,5 % et de préférence de 0,01 % à 0,2 % de kétorolac, et
(b) un excipient pour solution dentaire comprenant un conservateur, de préférence un conservateur de type parabenparahydroxybenzoate, et de l'eau.

6. Composition selon l'une quelconque des revendications 2 à 5, dans laquelle la composition a un pH de 4 à 7.

7. Composition selon l'une quelconque des revendications 2 à 6, dans laquelle le kétorolac est la kétorolac-trométhamine.

8. Composition selon l'une quelconque des revendications 2 à 7, dans laquelle le kétorolac est essentiellement constitué de l'énantiomère (-) ou S.

9. Utilisation de kétorolac selon la revendication 1 ou 2, dans laquelle le médicament se présente sous une forme choisie parmi les pâtes dentifrices, les gels dentifrices, les poudres dentifrices, les bains de bouche, les pulvérisations buccales, les pâtes prophylactiques et les solutions de traitement dentaire.

10. Utilisation de kétorolac selon la revendication 1, 2 ou 9, dans laquelle le médicament est maintenu dans la cavité buccale pendant un certain laps de temps, puis est ensuite largement expectoré plutôt qu'avalé.
